# FASCICULE DE BREVET EUROPEEN

(11) **EP 1 525 915 B1**
(45) Date de publication et mention de la délivrance du brevet: **03.03.2010**
(21) Numéro de dépôt: 04292430.8
(22) Date de dépôt: 12.10.2004
(51) Int. Cl.: B01J 29/76, B01J 29/78, C07C 6/12

(54) **Catalyseur comprenant au moins une zeolithe de type structural bog et son utilisation en transalkylation d'hydrocarbures alkylaromatiques**
Katalysator enthaltend mindestens einen Zeolith des BOG Strukturtyps und seine Verwendung zur Transalkylierung von alkylaromatischen Kohlenwasserstoffen
Catalyst comprising at least one BOG type zeolite and its use in the transalkylation of alkylaromatic hydrocarbons

(30) Priorité: 24.10.2003 FR 0312552
(43) Date de publication de la demande: 27.04.2005
(73) Titulaire: Institut Français du Pétrole, 92852 Rueil Malmaison Cedex (FR)
(72) Inventeur: Lacombe, Sylvie, 69230 Saint Genis Laval (FR); Guillon, Emmanuelle, 69230 Saint Genis Laval (FR)

(56) Documents cités:
- EP-A- 1 077 083
- FR-A- 2 821 075
- US-A- 5 952 536
- J.J. PLUTH AND J.V. SMITH: "Crystal structure of boggsite, a new high-silica zeolite with the first three-dimensional channel system bound by both 12- and 10- rings" AMERICAN MINERALOGIST., vol. 75, 1990, pages 501-507, XP008029889 WASHINGTON, DC, US, US ISSN: 0003-004X
- ADAIR BRIAN ET AL: "Reactions of meta-xylene on zeolites with intersecting medium and large pores. I. Basic studies" MICROPOROUS MATERIALS, vol. 7, no. 5, novembre 1996 (1996-11), pages 261-270, XP002277188 AMSTERDAM, NETHERLANDS

## Description

### Domaine technique :

La présente invention se rapporte à un nouveau catalyseur utilisable par exemple dans les réactions de transformation des hydrocarbures aromatiques. De manière plus précise, elle se rapporte à un catalyseur utilisé pour la transalkylation d'hydrocarbures alkylaromatiques et de préférence la transalkylation du toluène et de composés aromatiques contenant au moins 9 atomes de carbone, pour produire des xylènes. La présente invention concerne aussi la préparation dudit catalyseur ainsi que son utilisation dans un procédé de transalkylation d'hydrocarbures alkylaromatiques.

### Etat de la technique antérieure :

De nombreux catalyseurs de dismutation et/ou de transalkylation ont déjà été décrits dans l'art antérieur, et sont à base de mordénite (US-A-3.506.731, US-A-4.151.120, US-A-4.180.693, US-A-4.210.770, US-A-3.281.483, US-A-3.780.121, ou US-A-3.629.351), à base de zéolithe oméga (US-A-5.210.356, US-A-5.371.311 ) ou bien aussi à base de zéolithe de type structural NES (EP-A-1.077.083). Ces catalyseurs ne permettent pas de conduire à des performances catalytiques optimales, notamment en terme de sélectivité, dans les réactions de transalkylation d'hydrocarbures alkylaromatiques.
La présente invention se propose de fournir un nouveau catalyseur conduisant dans les réactions de transalkylation d'hydrocarbures alkylaromatiques à des performances catalytiques améliorées par rapport aux catalyseurs de l'art antérieur.

### Résumé

Le catalyseur de la présente invention contient au moins une zéolithe de type structural BOG, en particulier la Boggsite, comprenant du silicium et au moins un élément T choisi dans le groupe formé par l'aluminium, le fer, le gallium et le bore. Cette zéolithe est présente au moins en partie sous forme acide. Le liant est de préférence l'alumine. Le catalyseur contient en outre au moins un métal choisi dans le groupe constitué par les métaux des groupes VIB, VIIB et VIII non noble de la classification périodique des éléments. Enfin, éventuellement, le catalyseur contient, en plus, au moins un métal choisi dans le groupe formé par les éléments des groupes IIIA et IVA de la classification périodique des éléments. La présente invention concerne également l'utilisation du catalyseur dans un procédé de transalkylation d'hydrocarbures alkylaromatiques tels que le toluène et les alkylaromatiques ayant au moins 9 atomes de carbone. Ce catalyseur est en particulier très performant dans le traitement de charges aromatiques C9+ contenant un pourcentage de molécules aromatiques ayant au moins 10 atomes de carbone supérieur à 5% poids, cette charge pouvant également contenir du benzène.

### Description

L'invention concerne un catalyseur comprenant au moins un liant, au moins une zéolithe de type structural BOG, présente au moins en partie sous forme acide et comprenant du silicium et au moins un élément T choisi dans le groupe formé par l'aluminium, le fer, le gallium et le bore, ledit catalyseur comprenant en outre au moins un métal choisi dans le groupe constitué par les métaux des groupes VIB, VIIB et VIII non noble de la classification périodique des éléments.

La zéolithe de type structural BOG, en particulier la Boggsite, comprise dans le catalyseur selon l'invention a les caractéristiques de celle décrite dans "Atlas of Zeolite Framework Types", W. M Meier, D. H. Olson and Ch. Baerlocher, 5th revised edition, 2001, Elsevier auquel se réfère la présente demande.

La composition chimique de ladite zéolithe de type structural BOG telle que décrite dans l'''Atlas of Zeolite Framework Types" est : Na⁺₄Ca²+₇ (H₂O)₇₄ [Al₁₈Si₇₈O₁₉₂]. Ladite zéolithe de type structural BOG est caractérisée par le fait qu'elle présente un diagramme de diffraction de rayons X comportant au moins les raies présentées dans le tableau 1 (Pluth J. and Smith J. ; American Mineralogist 75 (1990) 501-507).

**Tableau 1**

| 2θ | Intensité I/Imax |
|---|---|
| 7.43 | faible |
| 7.84 | forte |
| 8.17 | faible |
| 8.74 | faible |
| 11.05 | faible |
| 13.13 | faible |
| 15.72 | faible |
| 18.02 | faible |
| 19.90 | moyenne |
| 20.38 | moyenne |
| 21.09 | faible |
| 22.19 | faible |
| 22.41 | faible |
| 23.05 | forte |
| 23.68 | faible |
| 24.10 | faible |
| 24.25 | faible |
| 24.69 | moyenne |
| 25.29 | faible |
| 26.32 | forte |
| 26.44 | forte |
| 26.99 | faible |
| 27.88 | faible |
| 27.89 | faible |
| 28.96 | faible |
| 29.28 | faible |
| 30.04 | faible |
| 31.10 | faible |
| 31.17 | faible |
| 32.23 | faible |
| 33.59 | faible |
| 34.05 | moyenne |
| 34.64 | faible |
| 37.29 | faible |
| 44.79 | faible |
| 45.72 | faible |
| 45.75 | faible |
| 46.55 | faible |

Elle se trouve à l'état naturel, notamment dans l'Etat d'Oregon aux USA.

La zéolithe de type structural BOG, en particulier la Boggsite, possède un réseau microporeux bidimensionnel constitué de canaux dont l'ouverture de pore est délimitée par des ouvertures à 10 et 12 atomes d'oxygène. Une ouverture de pore à 10 ou 12 atomes d'oxygène correspond à des pores de 10 ou 12 côtés. La détermination du diamètre de pores présents dans la zéolithe de type structural BOG a conduit aux valeurs suivantes: 7,0 x 7,0 Å pour les pores à 12 côtés et 5,5 x 5,8 Å pour les pores à 10 côtés.

Lorsqu'elle est comprise dans le catalyseur selon l'invention, la zéolithe de type structural BOG est au moins en partie, de préférence pratiquement totalement, sous forme acide, c'est-à-dire sous forme hydrogène (H⁺). La teneur en cations alcalin(s) et/ou alcalino-terreux est inférieure à 0,2 % poids, de préférence inférieure à 0,05 % poids par rapport au poids total de zéolithe sèche.

Le rapport Si/T atomique global de la zéolithe de type structural BOG présente dans le catalyseur selon l'invention est compris entre 4 et 100, de préférence entre 4 et 75 et de manière très préférée entre 4 et 50.

Le catalyseur selon l'invention contient au moins une zéolithe de type structural BOG à raison de 10 à 90 %, de préférence de 20 à 85% et de manière encore plus préférée de 50 à 85 % poids et au moins une matrice (ou liant) en complément à 100 % du catalyseur, ladite zéolithe comprenant du silicium et au moins un élément T choisi dans le groupe formé par l'aluminium, le fer, le gallium et le bore, de préférence l'aluminium.

Ledit catalyseur comprend en outre au moins un métal choisi dans le groupe constitué par les métaux des groupes VIB, VIIB et VIII non noble. Ledit métal est avantageusement présent à une teneur comprise entre 0,01 et 5 % et de préférence entre 0,05 et 3 % poids. Selon l'invention, le métal du groupe VIIB préféré est le rhénium, le métal du groupe VIB préféré est le molybdène et le métal préféré du groupe VIII non noble est le nickel. Avantageusement, le métal choisi dans le groupe constitué par les métaux des groupes VIB, VIIB et VIII non noble est le rhénium. Le catalyseur selon l'invention comprend éventuellement au moins un élément choisi dans l'ensemble formé par les groupes IIIA et IVA de la classification périodique des éléments, de préférence choisi dans le groupe formé par l'indium et l'étain, à une teneur comprise entre 0,01 et 5 % et de préférence entre 0,5 et 3 % poids.

La matrice, présente à une teneur comprise entre 10 et 90 %, de préférence entre 15 et 80 % poids et de manière encore plus préférée entre 15 et 50 % poids par rapport au poids total de catalyseur, est choisie généralement parmi les éléments du groupe formé par les argiles (par exemple parmi les argiles naturelles telles que le kaolin ou la bentonite), la magnésie, les alumines, les silices, l'oxyde de titane, l'oxyde de bore, la zircone, les phosphates d'aluminium, les phosphates de titane, les phosphates de zirconium, les silice-alumines et le charbon, de préférence parmi les éléments du groupe formé par les alumines et les argiles, de manière encore plus préférée parmi les alumines.

Un catalyseur préféré selon l'invention comprend en poids par rapport au poids total dudit catalyseur de 10 à 90 % poids de zéolithe de type structural BOG, de 0,01 à 5 % poids d'au moins un métal choisi dans le groupe constitué par les métaux des groupes VIB, VIIB et VIII non noble et au moins une matrice en complément à 100 %.

La zéolithe de type structural BOG est par exemple présente dans le catalyseur selon l'invention sous forme naturelle échangée. Elle peut aussi avoir été soumise à un traitement consistant à extraire une partie de l'élément T de la charpente zéolithique. Lorsque l'élément T est l'aluminium, on parle de désalumination. De préférence, deux méthodes de désalumination peuvent être employées mais toute autre méthode connue de l'Homme du métier entre aussi dans le cadre de l'invention.

Ces méthodes de désalumination décrites ci-après pour l'aluminium peuvent également être valables pour les autres éléments T.

La première méthode dite de l'attaque acide directe comprend une étape de traitement par une solution aqueuse d'un acide minéral tel que HNO₃ ou HCl ou organique tel que CH₃CO₂H. Cette dernière étape peut être répétée autant de fois qu'il est nécessaire afin d'obtenir le niveau de désalumination voulu. Préalablement à l'étape d'attaque acide directe, il est possible de réaliser un ou plusieurs échange(s) ionique(s) par au moins une solution NH₄NO₃ par exemple, de manière à éliminer au moins en partie, de préférence pratiquement totalement, les cations alcalins et/ou alcalino-terreux. De même, à la fin du traitement de désalumination par attaque acide directe, il est éventuellement possible de réaliser un ou plusieurs échange(s) ionique(s) par au moins une solution NH₄NO₃, de manière à éliminer les cations alcalin(s) et/ou alcalino-terreux résiduels et en particulier le sodium.

Pour atteindre le rapport Si/Al désiré, il est nécessaire de bien choisir les conditions opératoires ; de ce point de vue les paramètres les plus critiques sont la température de traitement par la solution aqueuse d'acide, la concentration dudit acide, la nature dudit acide, le rapport entre la quantité de solution acide et la masse de zéolithe traitée, la durée de traitement et le nombre de traitements réalisés.

On peut également effectuer des traitements de désalumination par des composés chimiques désaluminants tels que (à titre d'exemples et de façon non exhaustive) le tétrachlorure de silicium (SiCl₄), l'hexafluorosilicate d'ammonium [(NH4)₂SiF₆], l'acide éthylènediaminetétracétique (EDTA) ainsi que ses formes mono et disodique. Ces réactifs peuvent être utilisés en solution ou en phase gaz par exemple dans le cas de SiCl₄.

La deuxième méthode de désalumination dite de traitement thermique (en particulier à la vapeur d'eau ou "steaming") suivi d'une attaque acide, comprend, dans un premier temps une étape dans laquelle le solide est soumis à un ou plusieurs échange(s) ionique(s) par au moins une solution NH₄NO₃ par exemple, de manière à éliminer au moins en partie, de préférence pratiquement totalement, les cations alcalins et/ou alcalino-terreux, en particulier le sodium, présents en position cationique dans la zéolithe. La zéolithe ainsi obtenue est soumise à au moins un cycle de désalumination de charpente, comportant au moins un traitement thermique réalisé, éventuellement et de préférence en présence de vapeur d'eau, à une température généralement comprise entre 500 et 900°C, et éventuellement suivie d'au moins une attaque acide par une solution aqueuse d'un acide minéral ou organique. Les conditions de calcination en présence de vapeur d'eau (température, pression de vapeur d'eau et durée du traitement) ainsi que les conditions d'attaque acide post-calcination (durée de l'attaque, concentration de l'acide, nature de l'acide utilisé et rapport entre le volume d'acide et la masse de zéolithe) sont adaptées de manière à obtenir le niveau de désalumination souhaité. Dans le même but on peut aussi jouer sur le nombre de cycles traitement thermique - attaque acide qui sont effectués.

Une variante de cette deuxième méthode peut consister à remplacer l'étape dite de l'attaque acide, c'est-à-dire du traitement par une solution d'acide, par un traitement par une solution d'un composé chimique désaluminant tels que, par exemple, ceux cités précédemment à savoir le tétrachlorure de silicium (SiCl₄), l'hexafluorosilicate d'ammonium [(NH4)₂SiF₆], l'acide éthylènediaminetétracétique (EDTA) ainsi que ses formes mono et disodique.

Dans le cas préféré où l'élément T est l'aluminium, le cycle de désalumination de la charpente, comportant au moins une étape de traitement thermique réalisé, éventuellement et de préférence, en présence de vapeur d'eau et au moins une étape d'attaque en milieu acide de la zéolithe de type structural BOG, peut être répété autant de fois qu'il est nécessaire pour obtenir la zéolithe de type structural BOG désaluminée possédant les caractéristiques désirées. De même, suite au traitement thermique réalisé, éventuellement et de préférence en présence de vapeur d'eau, plusieurs attaques acides successives, avec des solutions en acide de concentrations différentes, peuvent être opérées.

Une variante de cette deuxième méthode de désalumination comprend le traitement thermique de la zéolithe de type structural BOG à une température généralement comprise entre 500°C et 900°C, éventuellement et de préférence en présence de vapeur d'eau. Puis, la zéolithe est éventuellement traitée par au moins une solution aqueuse d'un acide minéral (par exemple de HNO₃ ou de HCl) ou organique (CH₃CO₂H par exemple). Enfin, le solide ainsi obtenu peut être éventuellement soumis à au moins un échange ionique par au moins une solution NH₄NO₃ par exemple, de manière à éliminer pratiquement tout cation alcalin et/ou alcalino-terreux, en particulier le sodium, présent en position cationique dans la zéolithe.

Le catalyseur selon l'invention peut être préparé selon toute méthode connue de l'homme du métier. En général, il est obtenu par mélange de la matrice et de la zéolithe de type structural BOG, puis par mise en forme. Le métal choisi dans le groupe constitué par les métaux des groupes VIB, VIIB et VIII non-noble peut être introduit soit avant la mise en forme, ou bien lors du mélange, ou bien, et de préférence, après la mise en forme. On comprend ainsi que le mélange matrice + zéolithe est un support pour le catalyseur contenant le métal choisi dans le groupe constitué par les métaux des groupes VIB, VIIB et VIII non noble. La mise en forme est généralement suivie d'une calcination, généralement à une température comprise entre 250 et 600°C. Le métal choisi dans le groupe constitué par les métaux des groupes VIB, VIIB et VIII non noble peut être introduit après ladite calcination. Dans tous les cas, ledit métal est généralement déposé au choix soit pratiquement totalement sur la zéolithe, soit pratiquement totalement sur la matrice, soit en partie sur la zéolithe et en partie sur la matrice, ce choix s'effectuant de la manière qui est connue de l'homme du métier, par les paramètres utilisés lors dudit dépôt, comme par exemple la nature du précurseur choisi pour effectuer ledit dépôt.

Le métal choisi dans le groupe constitué par les métaux des groupes VIB, VIIB et VIII non noble, de préférence le rhénium, peut donc être déposé sur le mélange zéolithe-matrice préalablement mis en forme par tout procédé connu de l'homme du métier. Un tel dépôt est généralement effectué par la technique d'imprégnation à sec, d'échange(s) ionique(s) ou de coprécipitation. Parmi les précurseurs, on peut citer de manière non limitative, l'acide perrhénique et le perrhénate d'ammonium, déposés par exemple par imprégnation à sec.

Le dépôt du métal choisi dans le groupe constitué par les métaux des groupes VIB, VIIB et VIII non noble est suivi en général d'une calcination sous air ou oxygène, généralement entre 300 et 600°C, de préférence entre 350 et 550°C, et pour une durée comprise entre 0,5 et 10 heure(s), de préférence entre 1 et 4 heure(s).

Dans le cas où le catalyseur contient plusieurs métaux, ces derniers peuvent être introduits soit tous de la même façon soit par des techniques différentes, avant ou après mise en forme et dans n'importe quel ordre. Dans le cas où la technique utilisée est celle de l'échange ionique, plusieurs échanges successifs peuvent être nécessaires pour introduire les quantités requises de métaux.

La mise en forme du catalyseur selon l'invention est généralement telle que le catalyseur est sous forme de pastilles, d'agrégats, d'extrudés ou de billes, en vue de son utilisation, de préférence sous forme d'extrudés ou de billes.

Par exemple, une des méthodes préférées de préparation du catalyseur selon l'invention consiste à malaxer la zéolithe dans un gel humide de matrice (obtenu généralement par mélange d'au moins un acide et d'une poudre de matrice), par exemple d'alumine, pendant une durée nécessaire pour l'obtention d'une bonne homogénéité de la pâte ainsi obtenue, soit par exemple pendant une dizaine de minutes, puis à passer ladite pâte à travers une filière pour former des extrudés, par exemple de diamètre compris entre 0,4 et 4 mm. Puis, après séchage pendant quelques heures à 100 °C en étuve et après calcination, par exemple pendant 2 heures à 400 °C, le métal, par exemple le rhénium, peut être déposé, par exemple par imprégnation à sec d'une solution de perrhénate d'ammonium, ledit dépôt étant suivi d'une calcination finale, par exemple pendant 2 heures à 400°C. De manière préférée, le catalyseur obtenu est caractérisé par un coefficient de répartition macroscopique du métal, obtenu à partir de son profil déterminé par microsonde de Castaing, défini comme le rapport des concentrations dudit métal au coeur du grain par rapport au bord de ce même grain, compris de préférence entre 0,7 et 1,3, bornes incluses. De plus, de manière préférée, le catalyseur selon la présente invention, sous forme de billes ou d'extrudés, présente une valeur de l'écrasement en lit, déterminée selon la méthode Shell (SMS 1471-74) supérieure à 0,7 MPa.

La préparation du catalyseur se termine généralement par une calcination, dite calcination finale, habituellement à une température comprise entre 250 et 600°C, de préférence précédée d'un séchage, par exemple à l'étuve, à une température généralement comprise entre la température ambiante et 250°C, de préférence entre 40 et 200°C. Ladite étape de séchage est de préférence menée pendant la montée en température nécessaire pour effectuer ladite calcination.

On peut procéder ensuite à une réduction sous hydrogène, généralement à une température comprise entre 300 et 600°C, de préférence entre 350 et 550°C, et pour une durée comprise entre 1 et 10 heure(s), de préférence entre 2 et 5 heures. Une telle réduction peut avoir lieu *ex situ* ou *in situ,* par rapport au lieu d'utilisation dudit catalyseur dans une réaction donnée.

Le catalyseur de la présente invention peut éventuellement contenir du soufre. Dans ce cas, le soufre est introduit sur le catalyseur mis en forme, calciné, contenant le ou les élément(s) cité(s) précédemment, soit *in situ* avant la réaction catalytique, soit *ex situ.* La sulfuration s'effectue en utilisant tout agent sulfurant bien connu de l'homme du métier, tel que par exemple le disulfure de diméthyle ou le sulfure d'hydrogène. La sulfuration éventuelle intervient après la réduction. Dans le cas d'une sulfuration *in situ,* la réduction, si le catalyseur n'a pas été préalablement réduit, intervient avant la sulfuration. Dans le cas d'une sulfuration *ex situ,* on effectue la réduction puis la sulfuration.

Un autre objet de l'invention est l'utilisation du catalyseur selon l'invention contenant au moins une zéolithe de type structural BOG, en particulier la Boggsite, dans des procédés de conversion d'hydrocarbures.

L'invention concerne en particulier l'utilisation dudit catalyseur en transalkylation d'hydrocarbures alkylaromatiques, et de préférence la transalkylation du toluène et d'hydrocarbures alkylaromatiques généralement en C₉⁺ (c'est à dire à au moins 9 atomes de carbone par molécule), avec des mélanges toluène - AC₉⁺ (où AC₉⁺ désigne les hydrocarbures alkylaromatiques comportant au moins 9 atomes de carbone par molécule) pouvant contenir de 0 à 100 % de AC₉⁺ par rapport au mélange total. Ledit catalyseur se révèle très efficace pour ladite utilisation, car il se révèle être particulièrement actif, sélectif et stable, même en présence de charges à traiter contenant une grande quantité d'aromatiques lourds AC₉+, ces aromatiques lourds pouvant contenir une grande proportion d'AC₁₀⁺. Ainsi, des charges AC₉⁺ contenant au moins 5 % et jusqu'à 25 % poids, et même davantage d'AC₁₀⁺ peuvent être valorisées. A titre d'exemples, on peut citer de manière non exhaustive, les diméthyléthylbenzènes, les diéthylbenzènes, les propyléthylbenzènes... L'utilisation de ce catalyseur en transalkylation d'alkylaromatiques lourds est donc particulièrement intéressante.

Les conditions opératoires pour ladite utilisation sont généralement les suivantes : une température comprise entre 250 et 650°C et de préférence entre 350 et 550°C ; une pression comprise entre 1 et 6 MPa et de préférence entre 2 et 4,5 MPa ; une vitesse spatiale d'alimentation, exprimée en kilogramme de charge introduite par kilogramme de catalyseur et par heure, comprise entre 0,1 et 10 h⁻¹ et de préférence entre 0,5 et 4 h⁻¹ ; un rapport molaire hydrogène sur hydrocarbures compris entre 2 et 20 et de préférence entre 3 et 12 mol/mol.

### EXEMPLES

### Exemple 1 : Préparation d'un catalyseur à base de zéolithe Boggsite et de rhénium, conforme à l'invention.

La matière première utilisée est une zéolithe Boggsite (Oregon, USA) qui possède un rapport atomique Si/Al global égal à 4,3, et une teneur pondérale en cations (sodium, calcium) par rapport au poids en zéolithe Boggsite sèche d'environ 6,0 %.

Cette zéolithe Boggsite est soumise à quatre échanges ioniques successifs dans une solution de NH₄NO₃ 10N à environ 100°C pendant 4 heures de manière à retirer le sodium et la calcium résiduels. Entre chaque échange, la zéolithe est séchée une nuit à 100°C.

A l'issue de ces traitements, la zéolithe Boggsite sous forme H a un rapport Si/Al atomique global égal à 4,7, et une teneur pondérale en cations (sodium, calcium) par rapport au poids de zéolithe Boggsite sèche de 1100 ppm.

Cette zéolithe est ensuite mise en forme par extrusion avec un gel d'alumine de manière à obtenir, après séchage et calcination sous air sec, un support S1 qui contient en poids 70 % de zéolithe Boggsite forme H et 30 % d'alumine.

Le support S1 est ensuite imprégné à l'aide d'une solution aqueuse d'acide perrhénique, de manière à déposer 0,3 % poids de rhénium sur le solide. Le solide humide est ensuite séché à 120°C pendant 12 heures et calciné sous un débit d'air sec à la température de 500°C pendant une heure. Le catalyseur C1 ainsi obtenu contient en poids 69,7 % de Boggsite, 30,0 % d'alumine et 0,3 % de Rhénium.

### Exemple 2 : Préparation d'un catalyseur à base de zéolithe Boggsite et de nickel, conforme à l'invention.

On utilise dans cet exemple le support S1 décrit dans l'exemple 1 contenant en poids 70 % de zéolithe Boggsite forme H et 30 % d'alumine.
Le support S1 est soumis à une imprégnation à sec par une solution de nitrate de nickel de manière à déposer 0,3 % poids de nickel sur le solide. Le solide humide est ensuite séché à 120°C pendant 12 heures et calciné sous un débit d'air sec à la température de 500°C pendant une heure. Le catalyseur C2 ainsi obtenu contient en poids 69,8 % de Boggsite, 29,9 % d'alumine et 0,3 % de nickel.

### Exemple 3 : Préparation d'un catalyseur à base de zéolithe NU-87 et de rhénium, non conforme à l'invention.

La matière première utilisée est une zéolithe NU-87, qui possède un rapport atomique Si/Al global égal à 17,2, une teneur pondérale en sodium correspondant à un rapport atomique Na/Al égal à 0,14. Cette zéolithe NU-87 a été synthétisée d'après la demande de brevet européen EP-A-0.377.291 ou le brevet EP-B-0.378.916.

Cette zéolithe NU-87 subit, tout d'abord, une calcination dite sèche à 550°C sous flux d'air et d'azote durant 6 heures. Puis, le solide obtenu est soumis à un échange ionique dans une solution de NH₄NO₃ 10N, à environ 100°C pendant 4 heures. La zéolithe NU-87 est alors soumise à un traitement par une solution d'acide nitrique 7N, à environ 100°C, pendant 5 heures. Le volume V de la solution d'acide nitrique engagé (en ml) est égal à 10 fois le poids P de zéolithe NU-87 sèche (V/P=10). Ce traitement par une solution d'acide nitrique 7N est réalisé une seconde fois dans les mêmes conditions opératoires.

A l'issue de ces traitements, la zéolithe obtenue se trouve sous sa forme H et possède un rapport Si/Al atomique global égal à 34,6, et un rapport Na/Al égal à 0,007.

La zéolithe H-NU-87 est ensuite mise en forme par extrusion avec un gel d'alumine de manière à obtenir, après séchage et calcination sous air sec, le support S2 qui contient en poids 70 % de zéolithe H-NU-87 et 30 % d'alumine.

Le support S2 est imprégné à l'aide d'une solution aqueuse de perrhénate d'ammonium de manière à déposer 0,3 % poids de rhénium sur le solide. Le solide humide est ensuite séché à 120°C pendant 12 heures et calciné sous un débit d'air sec à la température de 500°C pendant une heure. Le catalyseur C3 ainsi obtenu contient en poids 69,8 % de NU-87 forme hydrogène, 29,9 % d'alumine et 0,31 % de rhénium.

### Exemple 4 : Comparaison des performances catalytiques des catalyseurs C1 et C3

Les catalyseurs sont préalablement réduits sous hydrogène à 450°C pendant 2 heures.

Les tests catalytiques ont été réalisés dans les conditions opératoires suivantes :
- température : 450°C
- pression totale : 3 MPa
- H2/HC : 5 mol/mol.

Une charge AC₉⁺, notée A1, contenant 83,0% poids d'aromatiques à 9 atomes de carbone et 17,0 % poids d'aromatiques à 10 atomes de carbone a été utilisée.

Les deux catalyseurs contenant du rhénium, l'un à base de zéolithe Boggsite (C1 conforme) et l'autre à base de zéolithe NU-87 (C3 non conforme) ont été comparés à iso-conversion avec une charge contenant 20 % de toluène et 80 % de charge A1. Les résultats sont présentés dans le tableau 2 :

**Tableau 2**

| Charge | 20 % Toluène / 80 % A1 | |
|---|---|---|
| Catalyseurs | C1 (conforme) | C3 (non conforme) |
| Conv. globale (%) | 54,9 | 54,9 |
| Rendements (%poids) | | |
| Légers C₁-C₄ | 11,6 | 12,2 |
| C₅⁺ | 0,8 | 1,1 |
| Benzène | 5,8 | 5,5 |
| Xylènes | 34,8 | 34,1 |
| Ethylbenzène | 0,9 | 0,7 |
| Lourds | 0,9 | 1,3 |

Ces résultats permettent de montrer la meilleure sélectivité du catalyseur à base de Boggsite. En effet, le rendement en xylènes est augmenté tandis que le rendement en C₅⁺, produits secondaires non désirés de la réaction, diminue. Certains des composés de cette fraction C₅⁺ pénalisent la pureté du benzène produit. De plus, cette fraction C₅⁺ contient essentiellement des paraffines à 5 et 6 atomes de carbone qui proviennent des cycles aromatiques (ouverture et craquage). Il y a donc perte de noyaux aromatiques accrue, et par suite perte en produits recherchés dans le cas du catalyseur C3 non conforme à l'invention.

## Revendications

1. Catalyseur comprenant au moins un liant, au moins une zéolithe de type structural BOG, présente au moins en partie sous forme acide, et comprenant du silicium et au moins un élément T choisi dans le groupe formé par l'aluminium, le fer, le gallium et le bore, ledit catalyseur comprenant en outre au moins un métal choisi dans le groupe constitué par les métaux des groupes VIB, VIIB et VIII non noble de la classification périodique des éléments.

2. Catalyseur selon la revendication 1 tel que le métal choisi dans le groupe constitué par les métaux des groupes VIB, VIIB et VIII non noble de la classification périodique des éléments est le rhénium.

3. Catalyseur selon la revendication 1 ou 2 tel que la zéolithe de type structural BOG est la zéolithe Boggsite.

4. Catalyseur selon l'une des revendications 1 à 3 tel que l'élément T est l'aluminium.

5. Catalyseur selon l'une des revendications 1 à 4 tel qu'il comprend au moins un élément choisi dans l'ensemble formé par les éléments des groupes IIIA et IVA de la classification périodique des éléments.

6. Catalyseur selon la revendication 5 tel que l'élément choisi dans l'ensemble formé par les éléments des groupes IIIA et IVA est l'indium ou l'étain.

7. Catalyseur selon l'une des revendications 1 à 6 tel qu'il comprend du soufre.

8. Catalyseur selon l'une des revendications 1 à 7 comprenant en poids par rapport au poids total de catalyseur de 10 à 90 % poids de zéolithe de type structural BOG, de 0,01 à 5 % poids d'au moins un métal choisi dans le groupe constitué par les métaux des groupes VIB, VIIB et VIII non noble et au moins une matrice en complément à 100 %.

9. Catalyseur selon l'une des revendications 1 à 8 comprenant de 0,01 à 5 % d'au moins un élément choisi dans l'ensemble formé par les éléments des groupes IIIA et IVA.

10. Utilisation du catalyseur selon l'une des revendications 1 à 9 dans des procédés de conversion d'hydrocarbures.

11. Utilisation selon la revendication 10 tel que ledit procédé est un procédé de transalkylation d'une charge d'hydrocarbures alkylaromatiques.

12. Utilisation selon la revendication 11 dans un procédé de transalkylation du toluène et d'hydrocarbures alkylaromatiques.

13. Utilisation selon la revendication 11 ou 12 pour le traitement de charges aromatiques contenant au moins 5 % poids d'aromatiques ayant au moins 10 atomes de carbone.

## Claims

1. Catalyst that comprises at least one binder, at least one BOG-structured zeolite, presented at least partially in acid form and that comprises silicon and at least one element T that is selected from the group that is formed by aluminum, iron, gallium and boron, whereby said catalyst also comprises at least one metal that is selected from the group that consists of the non-noble metals of groups VIB, VIIB and VIII of the periodic table.

2. Catalyst according to claim 1, such that the metal that is selected from the group that consists of the metals of groups VIB, VIIB and non-noble metals of group VIII of the periodic table is rhenium.

3. Catalyst according to claim 1 or 2, such that the BOG-structured zeolite is the Boggsite zeolite.

4. Catalyst according to one of claims 1 to 3, such that the element T is aluminum.

5. Catalyst according to one of claims 1 to 4, such that it comprises at least one element that is selected from among all the elements of groups IIIA and IVA of the periodic table.

6. Catalyst according to claim 5, such that the element that is selected from among all the elements of groups IIIA and IVA is indium or tin.

7. Catalyst according to one of claims 1 to 6, such that it comprises sulfur.

8. Catalyst according to one of claims 1 to 7 that comprises by weight, relative to the total weight of catalyst, 10 to 90% by weight of BOG-structured zeolite, 0.01 to 5% by weight of at least one metal that is selected from the goup that consists of the metals of groups VIB, VIIB and non-noble metals of group VIII, and at least one matrix made up to 100%.

9. Catalyst according to one of claims 1 to 8 that comprises 0.01 to 5% of at least one element that is selected from among all the elements of groups IIIA and IVA.

10. Use of the catalyst according to one of claims 1 to 9 in hydrocarbon conversion processes.

11. Use according to claim 10, such that said process is a process for transalkylation of an alkyl-aromatic hydrocarbon feedstock.

12. Use according to claim 11 in a process for transalkylation of toluene and alkyl-arotnatic hydrocarbons.

13. Use according to claim 11 or 12 for the treatment of aromatic feedstocks that contain at least 5% by weight of aromatic compounds having at least 10 carbon atoms.

## Patentansprüche

1. Katalysator, der mindestens ein Bindemittel, mindestens einen Zeolithen vom Strukturtyp BOG, der mindestens teilweise in Säureform vorhanden ist, Silicium und mindestens ein Element T, ausgewählt aus der Gruppe gebildet aus Aluminium, Eisen, Gallium und Bor umfasst, wobei der Katalysator ferner mindestens ein Metall, ausgewählt aus der Gruppe bestehend aus den Nichtedelmetallen der Gruppen VIB, VIIB und VIII des Periodensystems der Elemente umfasst.

2. Katalysator nach Anspruch 1, wobei das Metall, das aus der Gruppe bestehend aus den Nichtedelmetallen der Gruppen VIB, VIIB und VIII des Periodensystems der Elemente ausgewählt ist, Rhenium ist.

3. Katalysator nach Anspruch 1 oder 2, wobei der Zeolith vom Strukturtyp BOG der Zeolith Boggsit ist.

4. Katalysator nach einem der Ansprüche 1 bis 3, wobei das Element T Aluminium ist.

5. Katalysator nach einem der Ansprüche 1 bis 4, wobei er mindestens ein Element umfasst, das aus der Gesamtheit gebildet aus den Elementen der Gruppen IIIA und IVA des Periodensystems der Elemente ausgewählt ist.

6. Katalysator nach Anspruch 5, wobei das Element, das aus der Gesamtheit, gebildet aus den Elementen der Gruppen IIIA und IVA ausgewählt ist, Indium oder Zinn ist.

7. Katalysator nach einem der Ansprüche 1 bis 6, wobei er Schwefel umfasst.

8. Katalysator nach einem der Ansprüche 1 bis 7, der in Gewichtsprozent, bezogen auf das Gesamtgewicht des Katalysators 10 bis 90 Gew.-% Zeolith vom Strukturtyp BOG, 0,01 bis 5 Gew.-% mindestens eines Metalls, ausgewählt aus der Gruppe bestehend aus den Nichtedelmetallen der Gruppen VIB, VIIB und VIII und mindestens eine Matrix zur Ergänzung auf 100 % umfasst.

9. Katalysator nach einem der Ansprüche 1 bis 8, der 0,01 bis 5 % mindestens eines Elements umfasst, das aus der Gesamtheit gebildet aus den Elementen der Gruppen IIIA und IVA ausgewählt ist.

10. Verwendung des Katalysators nach einem der Ansprüche 1 bis 9 in Verfahren zur Umwandlung von Kohlenwasserstoffen.

11. Verwendung nach Anspruch 10, wobei das Verfahren ein Verfahren zur Transalkylierung einer Beschickung aus alkylaromatischen Kohlenwasserstoffen ist.

12. Verwendung nach Anspruch 11 in einem Verfahren zur Transalkylierung von Toluen und alkylaromatischen Kohlenwasserstoffen.

13. Verwendung nach Anspruch 11 oder 12 zur Behandlung von aromatischen Beschickungen, die mindestens 5 Gew.-% aromatische Verbindungen enthalten, die mindestens 10 Kohlenstoffatome haben.
